# EUROPÄISCHE PATENTSCHRIFT

(11) **EP 0 989 847 B1**
(45) Veröffentlichungstag und Bekanntmachung des Hinweises auf die Patenterteilung: **18.12.2002**
(21) Anmeldenummer: 98936092.0
(22) Anmeldetag: 04.06.1998
(51) Int. Cl.: A61K 9/127, A61K 49/00

(54) **MITTEL ZUR ANTITUMORTHERAPIE**
ANTI-TUMORAL THERAPY AGENT
AGENT POUR THERAPIE ANTITUMORALE

(30) Priorität: 06.06.1997 DE 19724796
(43) Veröffentlichungstag der Anmeldung: 05.04.2000
(73) Patentinhaber: MAX-DELBRÜCK-CENTRUM FÜR MOLEKULARE MEDIZIN, 13125 Berlin (DE)
(72) Erfinder: RESZKA, Regina, D-16341 Schwanebeck (DE); BERGER, Gerd, D-10785 Berlin (DE); POHLEN, Uwe, D-10625 Berlin (DE); JUNG, Marion, D-12055 Berlin (DE)
(74) Vertreter: Baumbach, Friedrich, Dr.
(86) Internationale Anmeldenummer: DE9801514
(87) Internationale Veröffentlichungsnummer: WO98055103

(56) Entgegenhaltungen:
- WO-A-98/07409
- DE-A- 4 341 478

## Beschreibung

Die Erfindung betrifft ein neues Mittel zur Antitumortherapie auf der Basis von liposomal verkapselten Cytostatika und/oder deren Metaboliten. Anwendungsgebiete der Erfindung sind die pharmazeutische Industrie und die Medizin.

Es sind mehrere Mittel für die Antitumortherapie bekannt. In DE 43 41 478 wurde ein Mittel beschrieben, das insbesondere zur Therapie nichtresektabler primärer und sekundärer Lebertumoren verwendet werden kann. Dieses Mittel enthält lyophilisierte Stärkepartikel, die mit einem oder mehreren Cytostatika kombiniert werden und in jod-, gadolinium- oder magnetithaltigen Kontrastmitteln gelöst sind. Ein bevorzugtes Cytostatikum für dieses Mittel ist Carboplatin.

Mit dem Mittel gemäß 43 41 478 wird eine hohe Konzentration des eingesetzten Cytostatikums im zu behandelnden Tumor erreicht. Ein Nachteil besteht jedoch darin, daß die Verweilzeit im Tumor nur ca. 4-6 Stunden beträgt, was für eine erfolgreiche Therapie im allgemeinen nicht ausreicht.

Die Erfindung hat das Ziel, ein Drug-Targeting zur Krebsbekämpfung durch geeignete Carriersysteme aufzubauen. Der Erfindung liegt die Aufgabe zugrunde, die Anreicherung von Cytostatika im Tumor und die Verweilzeit im Tumor deutlich zu erhöhen. Gleichzeitig sollen toxische Nebenwirkungen auf die übrigen Organe herabgesetzt werden.

Die Erfindung wird gemäß den Ansprüchen 1 bis 11 realisiert. Wesentlicher Bestandteil der Erfindung ist ist die Verkapselung der eingesetzten Cytostatika und/oder deren Metaboliten, bevorzugt mit PEG-Liposomen. Von großer Bedeutung ist ferner der Einsatz von abbaubaren Stärkepartikeln, was zu einer Flußverlangsamung führt und damit die Kontaktzeit erhöht.

Die Verkapselung der Cytostatika erfolgt in an sich bekannter Weise, z. B. durch Herstellung eines Gemischs von Eiphosphatidylcholin, Cholesterol, Dicetylphosphat und zusätzlich Polyethylenglycol in Chloroform und Diisopropylether.

Gegenstand der Erfindung sind auch die pharmazeutischen Zubereitungen des ersten Bestandteils des erfindungsgenäßen Mittels, die aus einem
a)natürlichen, halbsynthetischen oder vollsynthetischen Amphiphil wie Lipid, Tensid, Emulgator oder Polyethylenglycol(PEG) bzw. Lipid-PEG,
b)einem Steroid,
c)einer geladenen Lipidkomponente,
d)dem wasser- oder lipidlöslichen Cystatikum und
e)einer Trägerflüssigkeit und ggf. zusätzlichen Hilfsstoffen, z. B. Nanopartikeln besteht.
Das natürliche, halbsynthetische oder vollsynthetische Amphiphil hat bevorzugt die allgemeine Formel I 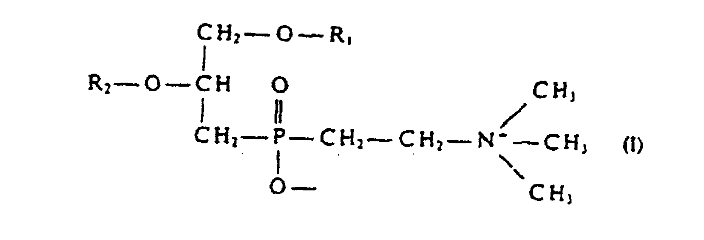 worin R₁ und R₂ = C₁₀-C₂₀-Alkanoyl, -Alkenoyl, Alkyl, -Alkenyl bedeuten.
Das Steroid hat bevorzugt die allgemeine Formel II 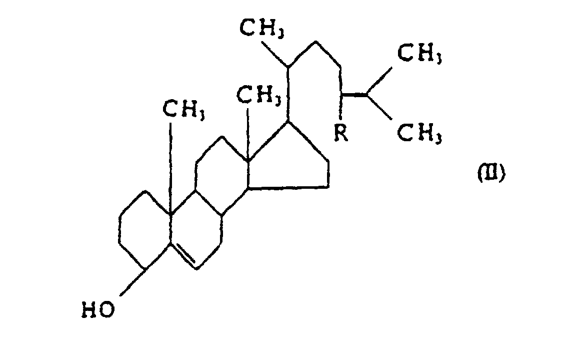 in der R = H (Cholesterol) oder = CH₂-CH₂-O-CH₂-CH₂-OH (Dicholesterol) bedeutet.
Die geladene Lipidkomponente ist bevorzugt das Anion des Dicetylphosphats, der Palaitinsäure, der Stearinsäure, das Anion eines Phospholipids, wie Phosphatidylserin, Phosphatidsäure oder das Anion eines Sphingolipids wie Sulfatid oder Polyethylenglycol wie MPES-DSPE.
Bevorzugte Cytostatika sind Carboplatin, 5-Fluoruracil und 5-Fluoruridin.
Die Mengenverhältnisse der Komponenten sind bevorzugt a:b:c im Molverhältnis 1:0,3:0,1 bis 1:1:0,1 oder bis 1:1:0:5 und c:d im Molverhältnis 2:1 bis 10:1.

Die Vorteile des neuen Mittels werden bei der Anwendung sichtbar. Sie liegen in der gegenüber den bekannten Mitteln wesentlich erhöhten Wirksamkeit, was dadurch begründet ist, daß eine größere Menge des Cytostatikums in den Tumor gebracht werden kann und dort für längere Zeit verweilt. Entscheidend für den therapeutischen Effekt ist der sog. AUC-Wert ("Area under the curve"), die Verweilzeit und die Menge des Therapeutikums, die im Tumor summiert wird. Dieser Wert ist bei Anwendung des erfindungsgemäßen Mittels deutlich höher als bei bekannten Mitteln, u. a. in dem Mittel gemäß DE 43 41 478. Aus Abb. 12 geht beispielsweise hervor, daß der AUC von verkapseltem 5-Fluoruridin um das 417fache gegenüber der freien Verbindung gesteigert ist (bei Zusatz von abbaubaren Stärkepartikeln um das 4,4 fache).

Von Bedeutung ist auch das Applikationsregime des erfindungsgemäßen Mittels. Die intraarterielle Applikation ergibt meist eine starke Erhöhung des AUC. Ein weiterer, für die praktische Anwendbarkeit wesentlicher Vorteil besteht darin, daß das Mittel auch oral angewendet werden kann.

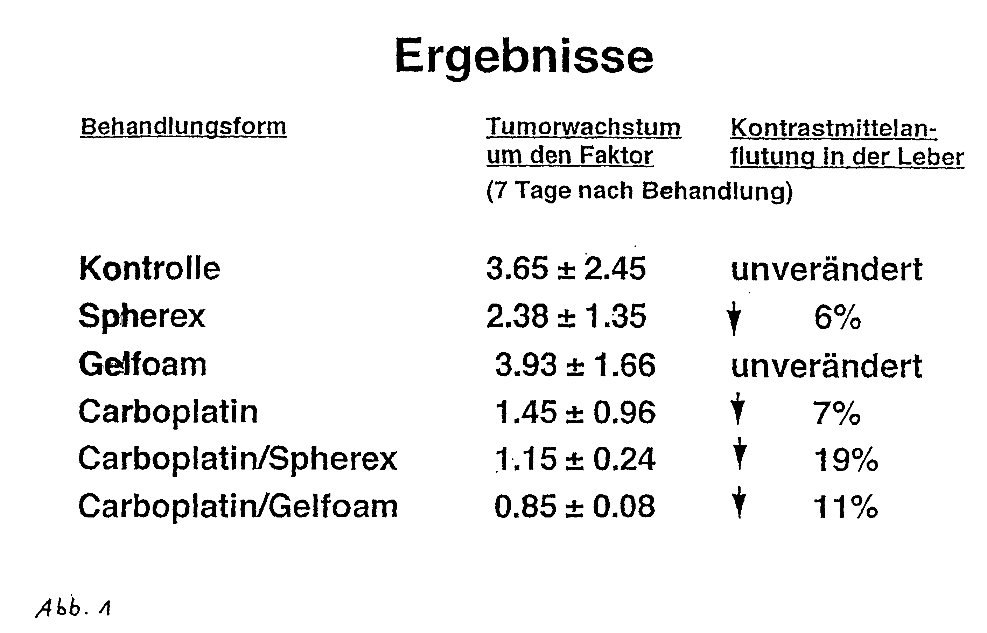

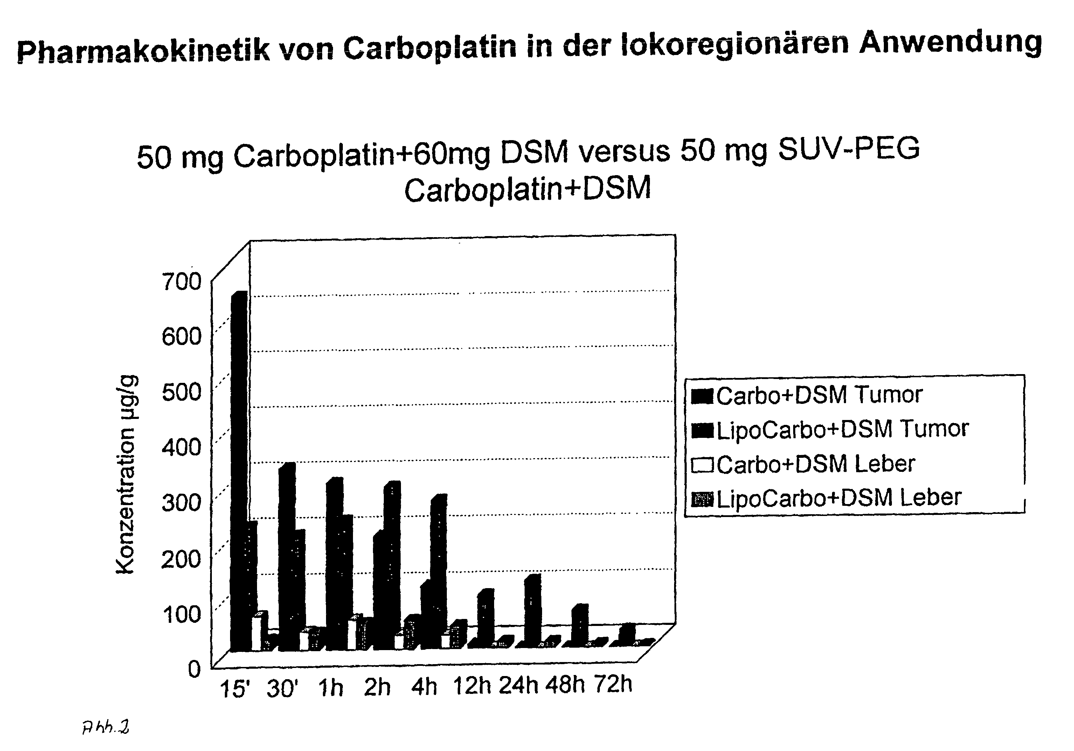

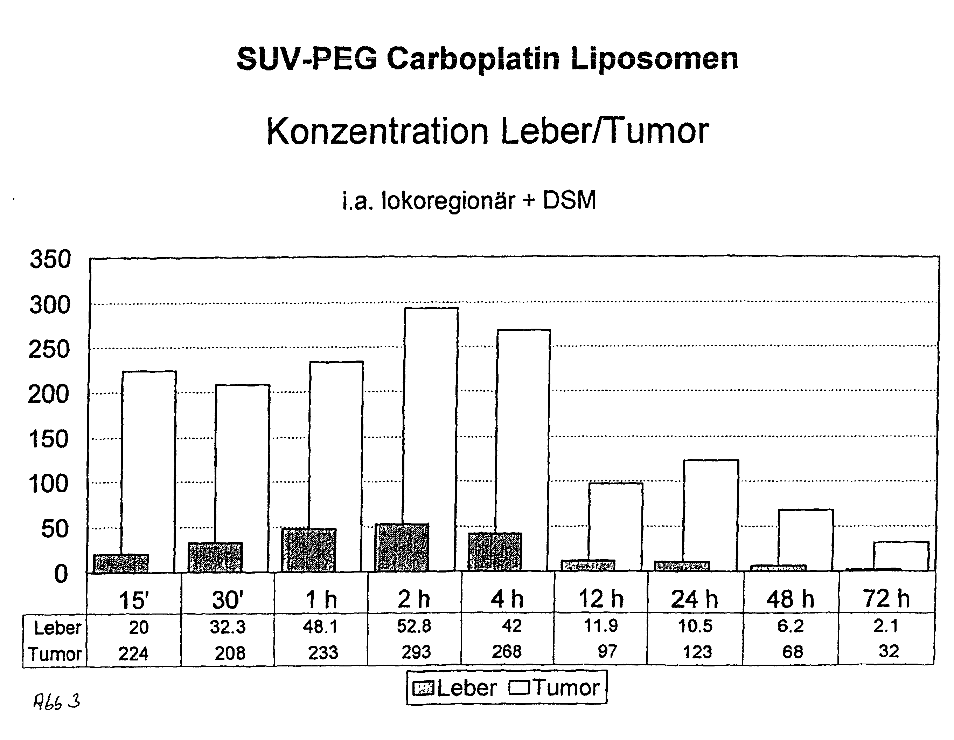

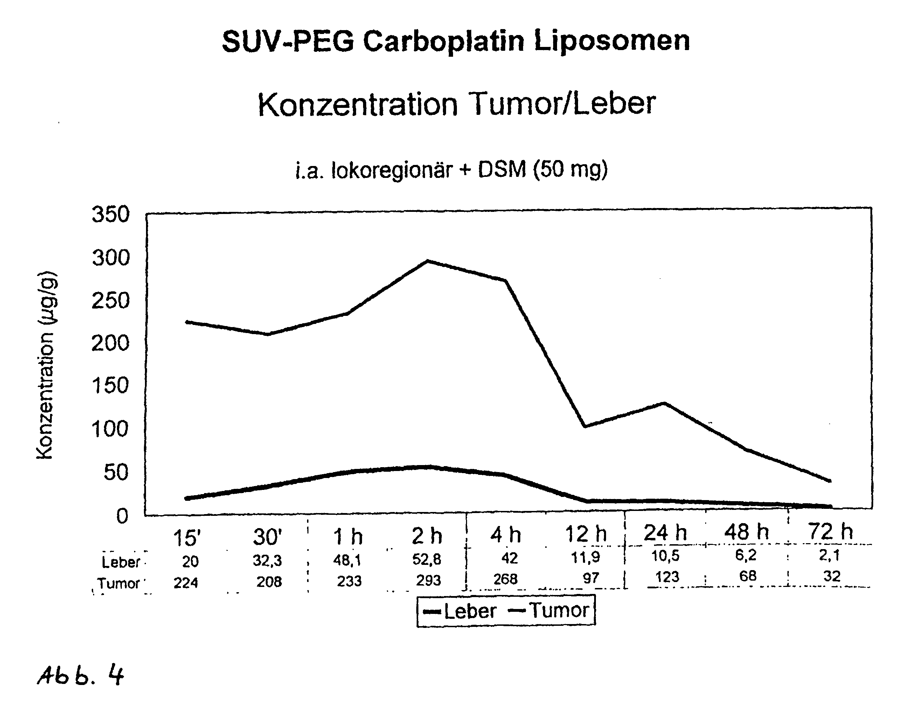

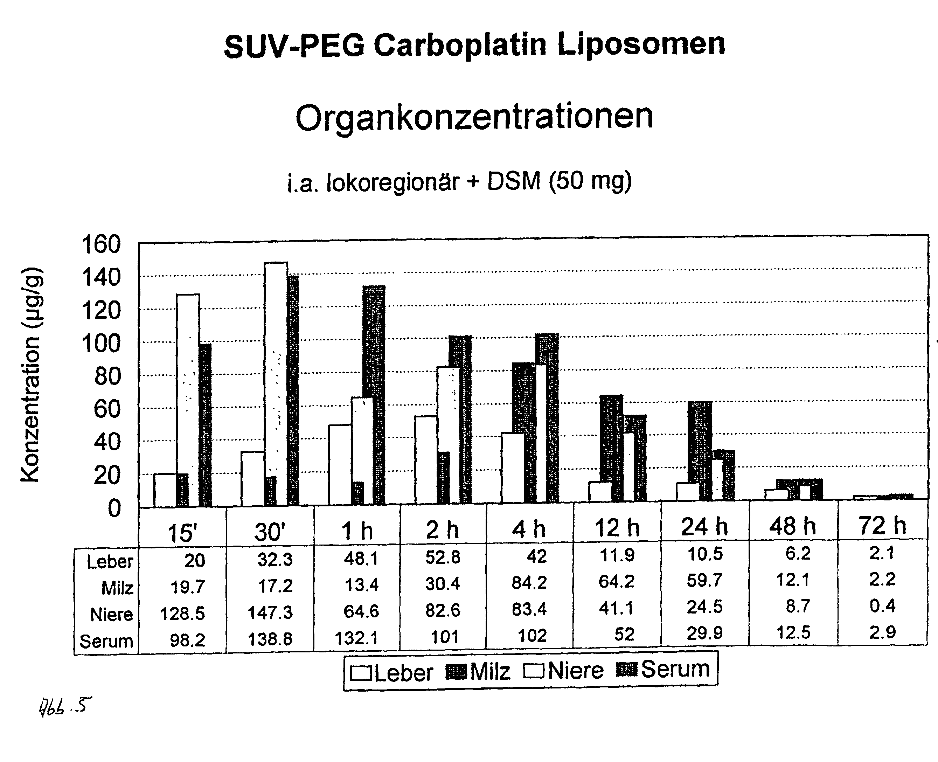

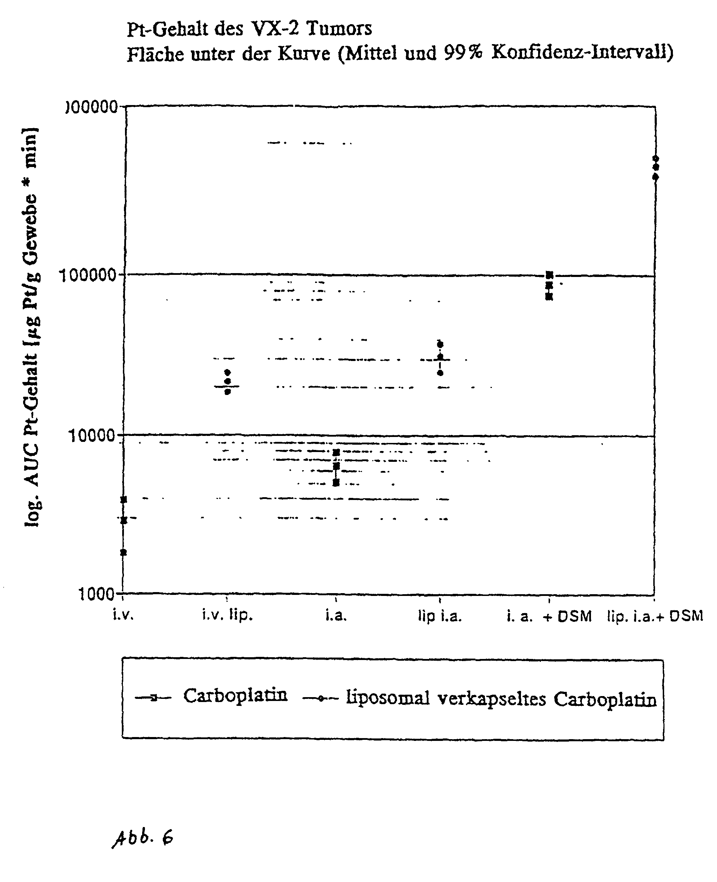

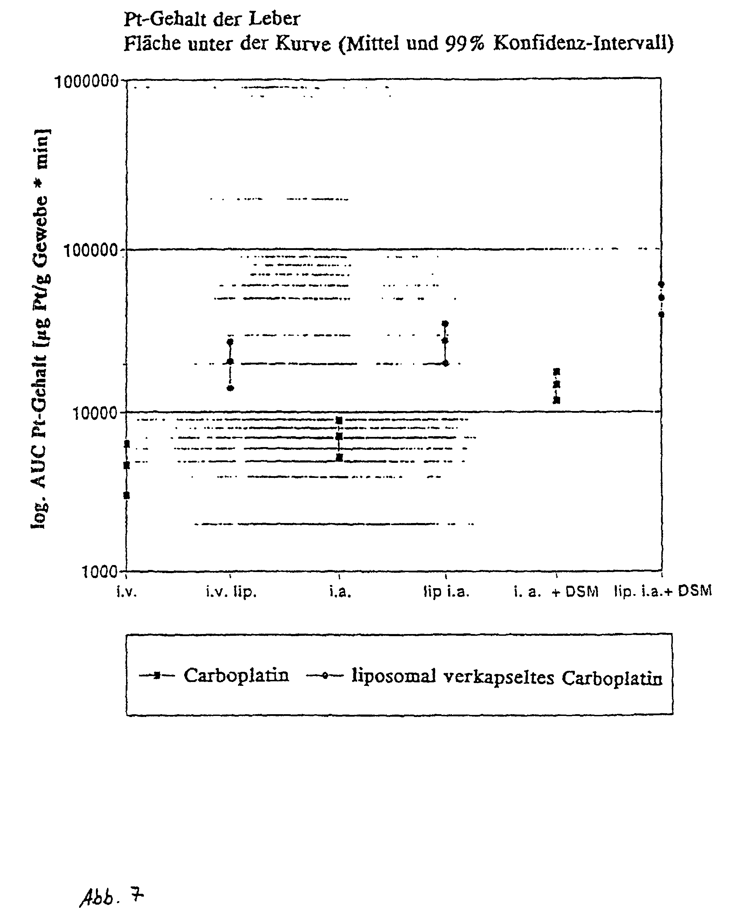

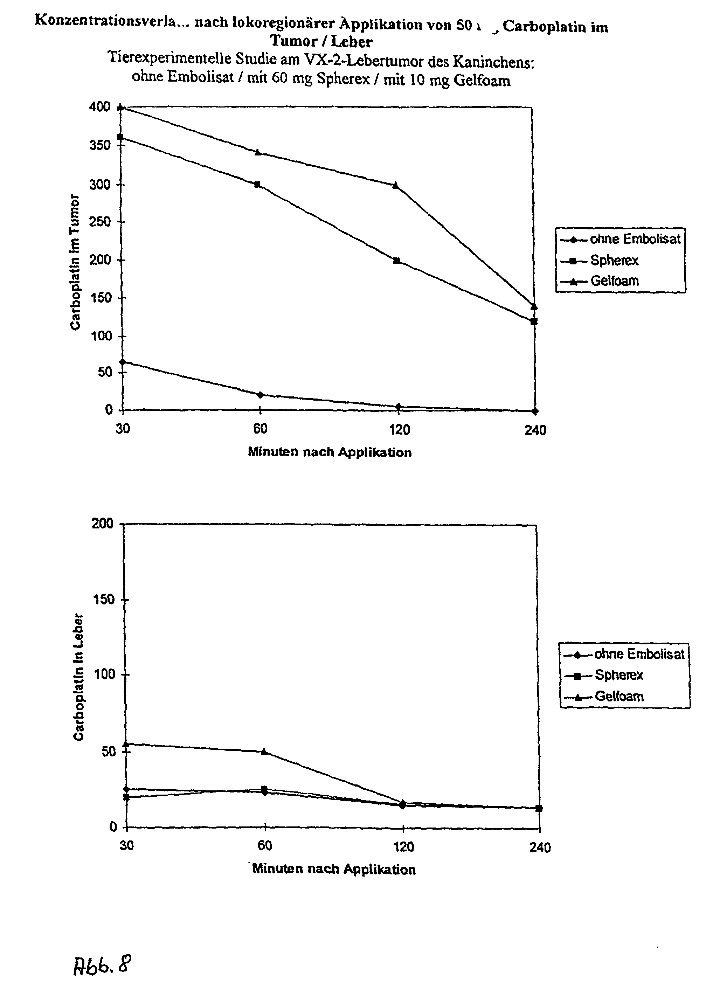

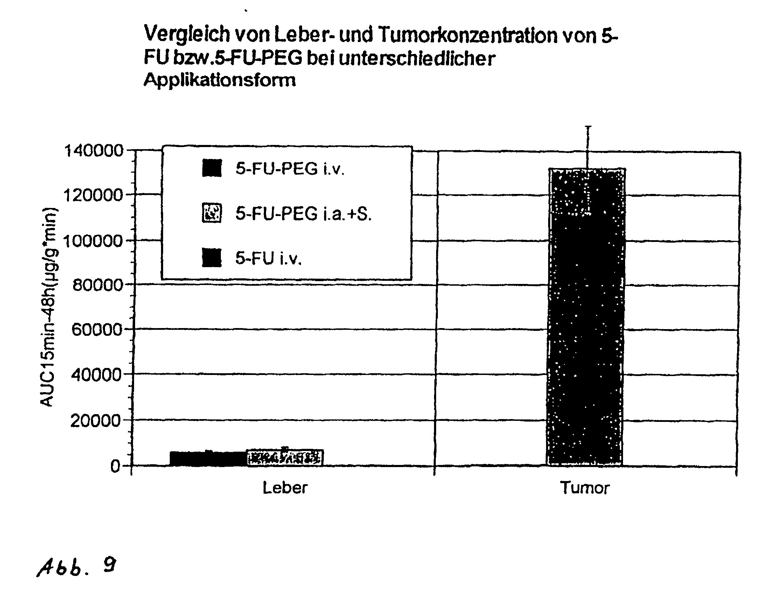

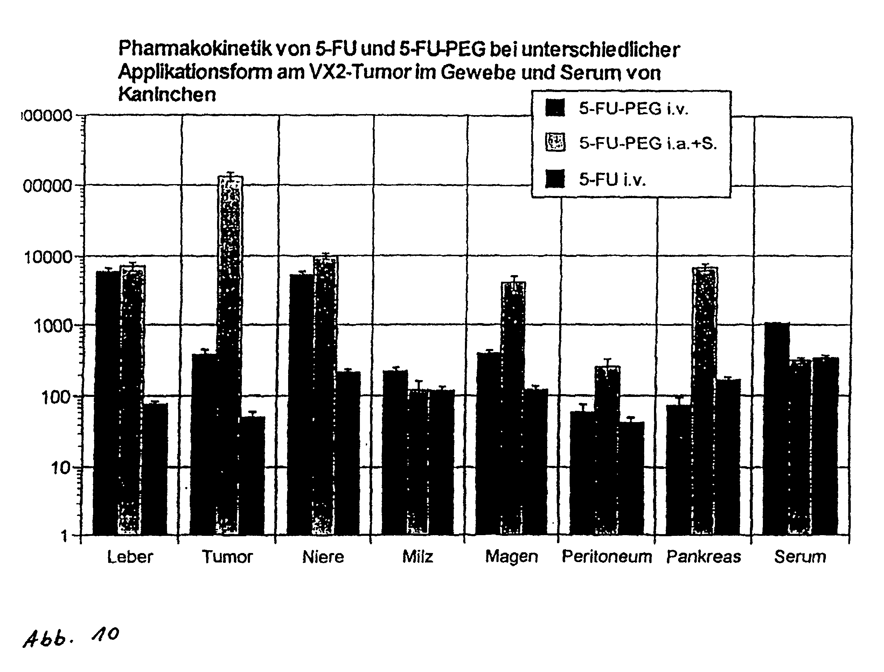

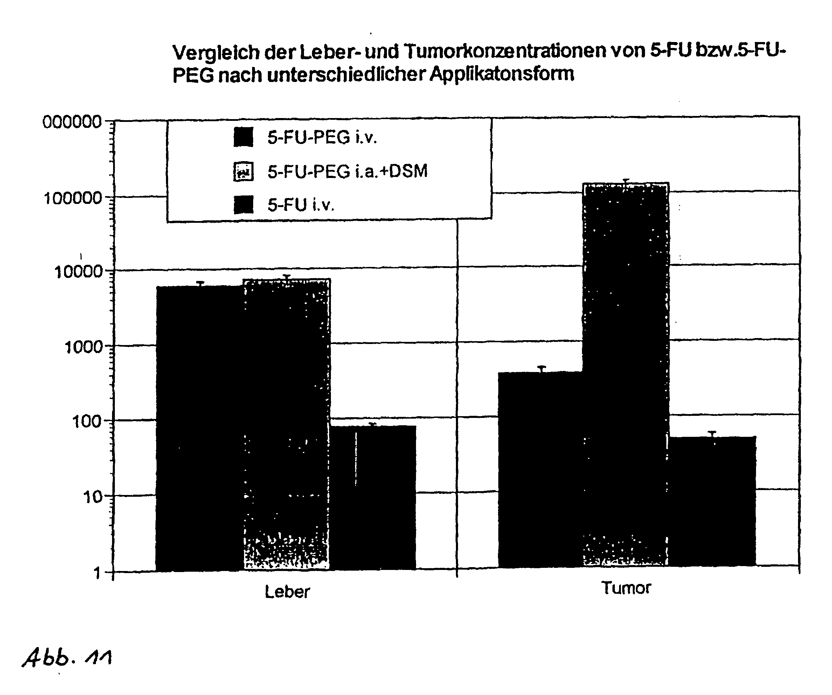

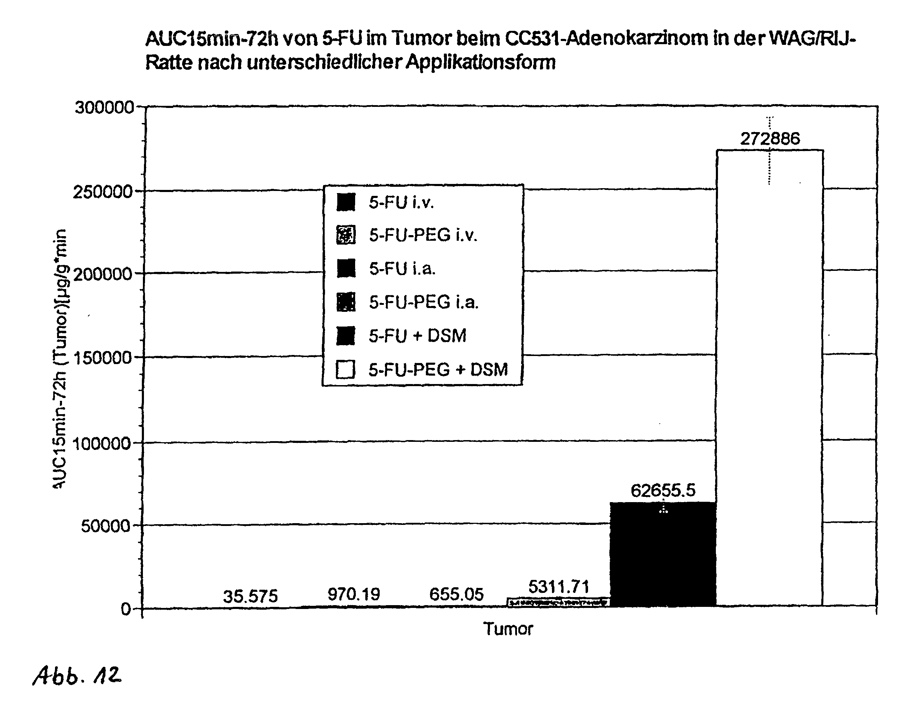

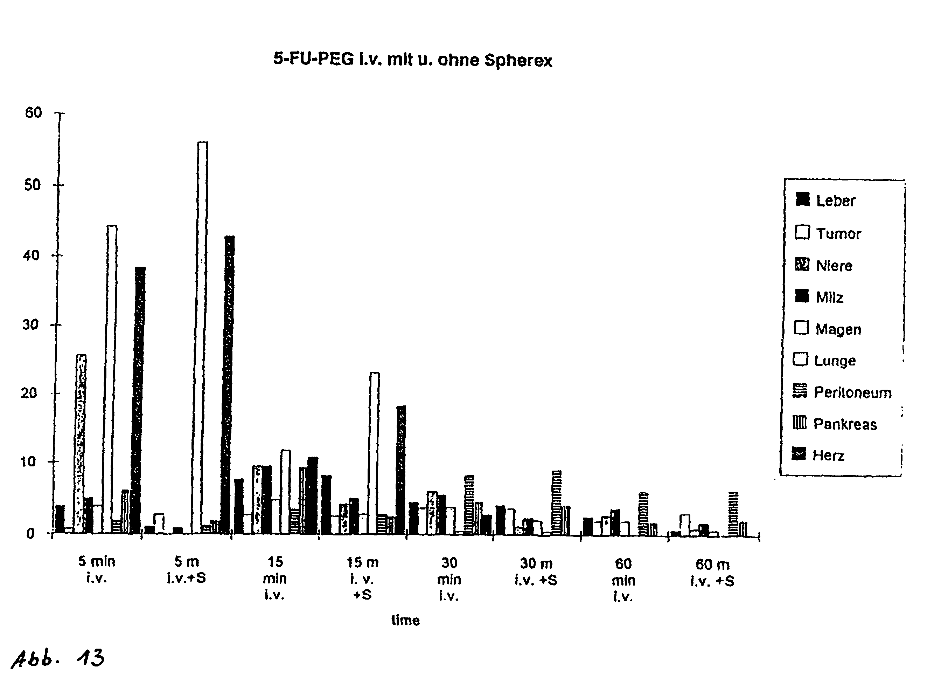

Die Erfindung wird durch Ausführungsbeispiele und nachfolgende Abbildungen 1 bis 13 näher erläutert.

### Beispiel 1:

Männlichen Chinchilla-Kaninchen werden 1 x 107 vitale VX2 Tumorzellen in den linken Leberlappen implantiert.
Bei Nachweis einer Tumorgröße von 2 cm erhielten die Tiere nach festgelegtem Schema entweder das erfindungsgemäße Therapeutikum oder ein Gemisch aus gleichen Dosen der handelsüblichen Form entweder als hepatic artery infusion (HAI) über das Portsystem oder intravenös. Dies beinhaltet jeweils 60 mg degradable starch microspheres (Spherex), 50 mg liposomal verkapseltes Carboplatin und 5 ml eines 300 mg/ml jodhaltigen Kontrastmittels (Ultravist 300, Schering). Zu den festgelegten Zeitpunkten (15, 30, 60, 120, 240 min, 8 Std., 12 Std., 24 Std., 48 Std.) wurden die Tiere getötet und die Zytostatikakonzentration im Tumor, Leber, Milz, Niere, Pankreas Magen Lymphknoten analytisch unter Verwendung der Atomabsorptionsspektroskopie ermittelt. Die AUC für das liposomale Carboplatin war im Tumor 20 fach erhöht.

### Beispiel 2:

Gleiches Vorgehen wie in Beispiel 1. Es wurden jedoch die in die Leber von WAG/Rij-Ratten implantierten CC 531 Adenocarcinome mit dem erfindungsgemäßen Therapeutikum behandelt. In diesem Modell wurden die Tiere mit 6 mg Spherex, 10 mg liposomalem 5-FU und 0,5 ml Ultravist behandelt. Zu den gleichen Zeitpunkten wie oben beschrieben wurden die Tiere getötet und die 5-FU-Konzentration und dessen Metabolie analytisch unter Verwendung der HLPC bestimmt. Die AUC für das liposomale 5-FU war im Tumor 20 fach erhöht.
Die Applikation des neu entwickelten Mittels konnte problemlos direkt unter Röntgenkontrolle beobachtet werden, wobei sich die allmächliche Aufsättigung des Tumorgefäßbettes bei stehenden Bildern von der Peripherie bis zum Gefäßstamm über die gesamte Phase der Embolisation dargestellt und während der gesamten Dauer des Gefäßverschlusses als stehendes Bild nachvollziehbar war. Ebenso konnte die einsetzende Reperfusion dokumentiert werden.

### Abbildungen

1. Ergebnisse mit Carboplatin als Cytostatikum, Verkapselung in SUV-PEG und Spherex bzw. Gelfoam als Stärkepartikel
   Abb. 1 Vergleich des Tumorwachstums
   Abb. 2-4 Pharmakokinetik in Tumor und Leber
   Abb. 5 Organkonzentrationn
   Abb. 6-7 Vergleich der Flächen unter der Kurve Carboplatin/liposomal verkapseltes Carboplatin
   Abb. 8 Tierexperimentelle Studie
2. Ergebnisse mit Fluoruracil
   Abb. 9 Konzentration in Tumor und Leber
   Abb. 10-12 Pharmakokinetik und Konzentrationen bei unterschiedlichen Applikationsformen
   Abb. 13 Applikation mit und ohne Spherex

## Patentansprüche

1. Mittel zur Antitumortherapie auf der Basis von liposomal verkapselten Cytostatika und/oder deren Metaboliten, enthaltend
- in PEG-, Immuno- oder Immuno/PEG-Liposomen verkapselte Cytostatika und/oder deren Metaboliten,
- abbaubare Stärkepartikel und/oder Gelatine und/oder Manopartikel
- jod-, gadolinium- oder magnetithaltige Kontrastmittel.

2. Mittel nach Anspruch 1, **dadurch gekennzeichnet, daß** die Verkapselung der Cytostatika und/oder deren Metaboliten in
- SUV(Small unilamellar vesicles)-PEG
- LUV(Large unilamellar vesicles)-PEG
- REV(Reversed face evaporation vesicles)-PEG
- MLV(Multilamellar vesicles)-PEG
erfolgt.

3. Mittel nach Anspruch 1, **dadurch gekennzeichnet, daß** die Verkapselung der Cytostatika und/oder deren Metaboliten in
- Anti-Ki-67-Immun-PEG-Liposomen
erfolgt.

4. Mittel nach Anspruch 1, **dadurch gekennzeichnet, daß** die Verkapselung der Cytostatika und/oder deren Metaboliten in
- Anti-CEA-PEG-Liposomen
erfolgt.

5. Mittel nach Anspruch 1, **dadurch gekennzeichnet, daß** die Stärkepartikel eine Korngröße von 60-90 nm aufweisen.

6. Mittel nach Anspruch 1, **dadurch gekennzeichnet, daß** absorbierbare Gelatinepuder eingesetzt werden.

7. Mittel nach Anspruch 1, **dadurch gekennzeichnet, daß** als Nanopartikel eine 25%ige wäßrige Lösung von Poloxamer eingesetzt wird.

8. Mittel nach Anspruch 1, gekennnzeichnet durch folgende Bestandteile
- als Cytostatikum 5-Fluoruracil,
- Verkapselung in SUV-PEG,
- als Stärkepartikel Spherex,
- als Kontrastmittel Gadolinium-DTPA.

9. Mittel nach Anspruch 1, gekennnzeichnet durch folgende Bestandteile
- als Cytostatikum 5-Fluoruridin,
- Verkapselung in SUV-PEG,
- als Stärkepartikel Spherex,
- als Kontrastmittel Gadolinium-DTPA.

10. Mittel nach Anspruch 1, gekennnzeichnet durch folgende Bestandteile
- als Cytostatikum Carboplatin,
- Verkapselung in SUV-PEG,
- als Stärkepartikel Spherex oder Gelfoam,
- als Kontrastmittel Gadolinium-DTPA.

11. Mittel nach Anspruch 1, gekennnzeichnet durch folgende Bestandteile
- als Cytostatikum 5-Fluoruridin-5'-hexadecylphosphat,
- Verkapselung in SUV-PEG,
- als Stärkepartikel Spherex oder Gelfoam,
- als Kontrastmittel Gadolinium-DTPA.

12. Mittel nach den Ansprüchen 1 bis 6 zur Verwendung als Arzneimittel durch intraartielle, intravenöse oder orale Applikation.

## Claims

1. Agent for anti-tumour therapy on the basis of liposomally encapsulated cytostatics and/or their metabolites, containing
- cytostatics and/or their metabolites encapsulated in PEG, immuno or immuno/PEG liposomes
- degradable starch particles and/or gelatines and/or nano-particles
- contrast agents containing iodine, gadolinium or magnetite.

2. Agent according to Claim 1, wherein the encapsulation of the cytostatics and/or their metabolites is done in
- SUV(small unilamellar vesicles)-PEG
- LUV(large unilamellar vesicles)-PEG
- REV(reversed face evaporation vesicles)-PEG
- MLV(multilamellar vesicles)-PEG.

3. Agent according to Claim 1, wherein the encapsulation of the cytostatics and/or their metabolites is done in
- anti-Ki-67 immuno-PEG liposomes.

4. Agent according to Claim 1, wherein the encapsulation of the cytostatics and/or their metabolites is done in
- anti-CEA-PEG liposomes.

5. Agent according to Claim 1, wherein the starch particles exhibit a diameter of 60-90 nm.

6. Agent according to Claim 1, wherein absorbable gelatine powders are used.

7. Agent according to Claim 1, wherein a 25% aqueous solution of poloxamer is used as nano-particles.

8. Agent according to Claim 1, wherein there are the following components:
- 5-fluorouracil as a cytostatic
- encapsulation in SUV-PEG
- Spherex as starch particles
- gadolinium-DTPA as a contrast agent.

9. Agent according to Claim 1, wherein there are the following components:
- 5-fluorouridine as a cytostatic
- encapsulation in SUV-PEG
- Spherex as starch particles
- gadolinium-DTPA as a contrast agent.

10. Agent according to Claim 1, wherein there are the following components:
- carboplatin as a cytostatic
- encapsulation in SUV-PEG
- Spherex or gel foam as starch particles
- gadolinium-DTPA as a contrast agent.

11. Agent according to Claim 1, wherein there are the following components:
- 5-fluorouridine-5'-hexadecylphosphate as a cytostatic
- encapsulation in SUV-PEG
- Spherex or gel foam as starch particles
- gadolinium-DTPA as a contrast agent.

12. Agent according to Claims 1 to 6 for use as a medication by intra-arterial, intravenous or oral application.

## Revendications

1. Produit destiné au traitement anti-tumeur sur la base de cytostatiques capsulés en véhicule liposomal et/ou de leurs métabolites, contenant
- des cytostatiques et/ou de leurs métabolites capsulés en liposomes PEG, immuno-liposomes ou immuno-liposomes/PEG,
- de particules d'amidon dégradables et/ou de gélatine et/ou de nanoparticules
- de substance de contraste contenant de l'iode, du gadolinium ou de la magnétite.

2. Produit selon la revendication 1, se caractérisant par le fait que le capsulage des cytostatiques et /ou de leurs métabolites se fait en
- SUV (Small unilamellar vesicles)-PEG (Petits liposomes unilamellaires-PEG)
- LUV (Large unilamellar vesicles)-PEG (Grands liposomes unilamellaires-PEG)
- REV (Reversed face evaporation vesicles)-PEG
- MLV (Multilamellar vesicles)-PEG (Liposomes multilamellaires-PEG).

3. Produit selon la revendication 1, se caractérisant par le fait que le capsulage des cytostatique et/ou de leurs métabolites se fait en
- liposomes anti-Ki-67-immun-PEG.

4. Produit selon la revendication 1, se caractérisant par le fait que le capsulage des cytostatique et/ou de leurs métabolites se fait en
- liposomes anti-CEA-PEG.

5. Produit selon la revendication 1, se caractérisant par le fait que les particules d'amidon présentent une grosseur de grain de 60-90 nm.

6. Produit selon la revendication 1, se caractérisant par le fait que l'on utilise de la poudre de gélatine absorbable.

7. Produit selon la revendication 1, se caractérisant par le fait que l'on utilise en tant que nanoparticule une solution à 25 % aqueuse de poloxamer.

8. Produit selon la revendication 1, se caractérisant par les composants suivants
- 5-fluoruouracil en tant que cytostatique,
- capsulage en SUV-PEG,
- Spherex en tant que particule d'amidon,
- Gadolinium-DTPA en tant que substance de contraste.

9. Produit selon la revendication 1, se caractérisant par les composants suivants
- 5-fluoruridine en tant que cytostatique,
- capsulage en SUV-PEG,
- Spherex en tant que particule d'amidon,
- Gadolinium-DTPA en tant que substance de contraste

10. Produit selon la revendication 1, se caractérisant par les composants suivants
- Carboplatine en tant que cytostatique,
- capsulage en SUV-PEG,
- Spherex ou Gelfoam en tant que particule d'amidon,
- Gadolinium-DTPA en tant que substance de contraste

11. Produit selon la revendication 1, se caractérisant par les composants suivants
- 5-fluoruridin-5'-hexadecylphosphate en tant que cytostatique,
- capsulage en SUV-PEG,
- Spherex ou Gelfoam en tant que particule d'amidon,
- Gadolinium-DTPA en tant que substance de contraste

12. Produit selon les revendications 1 à 6 pour l'emploi en tant que médicament par application intra-artérielle, intraveineuse ou orale.
